# EUROPEAN PATENT SPECIFICATION

(11) **EP 3 656 367 B1**
(45) Date of publication and mention of the grant of the patent: **14.10.2026**
(21) Application number: 20151768.7
(22) Date of filing: 21.04.2015
(51) Int. Cl.: A61J 1/20, A61J 1/14

(54) **FLUID TRANSFER DEVICE AND PACKAGING THEREFOR**
FLUIDTRANSFERVORRICHTUNG UND VERPACKUNG DAFÜR
DISPOSITIF DE TRANSFERT DE FLUIDE ET SON EMBALLAGE

(30) Priority: 21.04.2014 US 201461982049 P; 21.04.2014 US 201461982091 P; 16.09.2014 US 201462050930 P
(43) Date of publication of application: 27.05.2020
(60) Divisional application: 20179913.7 / 3 733 147
(62) Document number(s) of the earlier application(s) in accordance with Art. 76 EPC: 15721425.5 / 3 134 058
(73) Proprietor: Becton Dickinson and Company Limited, Dun Laoghaire (IE)
(72) Inventor: Sanders, Laurie, Glen Ridge, NJ 07028 (US); Kim, Jayeon, Little Ferry, NJ 07643 (US)
(74) Representative: dompatent

(56) References cited:
- WO-A1-2011/146012
- WO-A2-2006/058435

## Description

### BACKGROUND OF THE INVENTION

### Field of the Invention

The present invention relates to a fluid transfer device for a closed transfer of fluid from a medical device to a patient delivery device, such as an IV line or syringe.

### Description of Related Art

Healthcare workers, such as pharmacists and nurses, can be subject to acute and long term health risks upon repeated exposure to drugs or solvents which might escape into the air during drug preparation, drug administration, and other similar handling. This problem is particularly serious when cytotoxins, antiviral drugs, antibiotics, and radiopharmaceuticals are concerned. The health risks faced by exposure to these drugs can include the development of cancer, reproductive problems, genetic conditions, and other serious concerns. Other hazardous areas may be sample taking, such as samples concerning virus infections or the like. When performing infusions, it is often necessary to inject a drug or other medical substance into the infusion fluid, inside an infusion bag or other infusion fluid container. This is often done by means of penetrating a septum or other fluid barrier of an injection port on the infusion bag or on the infusion fluid line with a needle of a syringe filled with the medical fluid in question. However, even before this, it may be necessary to transfer the medical fluid from a vial to a syringe and then from the syringe to a secondary container. In each of these steps, staff may be exposed to the medical fluid by means of contamination. Such contamination may be vaporized medical fluid or aerosol in the air. The contaminations may contaminate the staff through their lungs, or by vaporized medical fluid or aerosol in the air which condensates on the skin to thereafter penetrate the skin of the staff. Some medicaments are even known to penetrate protection gloves and thereby contaminate the staff.

Exposure to contaminations like this may, on a long term basis, give rise to alarmingly high concentrations of medicaments in the blood or the human body of the staff as described above. It has been understood that, due to the many transferring steps between containers e.g., vials, syringes, infusion systems, etc., the risk for contamination during the actual insertion and retraction of a needle from the container, e.g., a vial, needs to be contained. Closed system transfer devices (CSTDs) have been developed to ensure that the medicament is contained in the transfer device during transfer of the medicament.

Generally, a CSTD includes an adapter for connection to a syringe and an adapter for connection to a vial, a second syringe, or a conduit providing fluid access to the patient's circulatory system. According to one arrangement, the healthcare practitioner may reconstitute a powdered or lyophilized compound with saline or some other reconstitution medium by attaching the syringe to the vial via connection of the respective adapters, reconstituting the drug, aspirating the compound into the syringe, disconnecting the adapters, and then attaching the syringe to the fluid conduit through the respective adapters to a patient delivery device, such as an IV line or syringe for administration to the patient.

One type of an adapter that can be used in a CSTD has a first connector having a male or female luer-lock element that is arranged to be joined with a corresponding female or male luer-lock element of a second connector component. According to one aspect, the second connector component can be a patient delivery device, such as an IV line or a syringe. The luer-lock element can, thus, be screwed into and unscrewed from the corresponding luer-lock element. It is desirable to prevent an accidental or inadvertent unscrewing of the components, which could lead to the disconnection of the fluid passage. Such disconnection may entail a serious contamination risk for a patient and/or any other person in the vicinity of the disconnected medical connector. The issue of safety in administration of hazardous medical compounds is one that has been identified as being of critical importance by professional organizations and government agencies alike.

It is, therefore, desirable to provide an adapter for enabling fluid transfer between the first connector and the second connector by facilitating a positive connection of the connectors and avoiding inadvertent or accidental disconnection of the connectors.

A fluid transfer device comprising an inner member, movable in an axial direction within an outer member surrounding at least a portion of the inner member, and a seal arrangement preventing the inner member between two axial positions is described in WO 2006/058435.

### SUMMARY OF THE INVENTION

A fluid transfer device according to the invention is defined by the features of claim 1. Preferred embodiments are defined by the features of the dependent claims.

A fluid transfer system includes packaging having a body with a sidewall extending between an open top end and a bottom end and defining an interior cavity, and at least one protrusion extending radially inward from the sidewall into the interior cavity. The system also includes a fluid transfer device configured to be received within the interior cavity and including an inner member configured to be secured to a container and an outer member surrounding at least a portion of the inner member. The inner member having a first position where the inner member is configured to rotate freely relative to the outer member and a second position where rotation of the inner member relative to the outer member is restricted in a first rotational direction. The fluid transfer device also including a locking arrangement provided on at least a portion of the inner member and accessible through at least a portion of the outer member. The locking arrangement is configured for cooperating with the at least one protrusion to prevent rotation of the inner member relative to the outer member upon an application of a compressive force on the packaging.

The at least one protrusion may be a pair of protrusions that are oriented opposite from each other around a circumference of the packaging. The packaging may further include at least one button extending radially outward from an outer portion of the sidewall opposite the at least one protrusion. The at least one protrusion may be configured to deflect radially inward in response to the compressive force directed to the at least one button.

The outer member of the fluid transfer device may define an opening configured to receive the at least one protrusion of the packaging when the fluid transfer device is positioned within the interior cavity of the packaging. The at least one protrusion of the packaging and the opening of the outer member of the fluid transfer device may cooperate to prevent rotation of the fluid transfer device relative to the packaging when the fluid transfer device is positioned within the interior cavity of the packaging.

The at least one protrusion of the packaging may be configured to engage the locking arrangement through the opening in the outer member of the fluid transfer device to prevent rotation of the inner member relative to the outer member when the fluid transfer device is positioned within the interior cavity of the packaging.

The locking arrangement may be a recess defined by the inner member, with the recess having an engagement surface configured to engage the at least one protrusion of the packaging. The inner member may be a luer connection.

The inner member and the outer member define a ratchet arrangement, where the ratchet arrangement is only engaged when the inner member is in the second position. The inner member is moveable in an axial direction within the outer member between the first and second positions of the inner member.

The packaging may define a plurality of ribs extending radially inward from the sidewall of the packaging, with the plurality of ribs configured to engage a portion of the fluid transfer device when the fluid transfer device is positioned within the interior cavity of the packaging.

The fluid transfer system further includes a seal arrangement positioned within the outer member, with the seal arrangement moveable within the outer member and configured to engage a mating connector. The seal arrangement having an actuated position adjacent to the inner member and an initial position spaced from the inner member, where the seal arrangement prevents the inner member from moving from the first position to the second position when the seal arrangement is in the actuated position. The inner member and the outer member define a ratchet arrangement, where the ratchet arrangement is only engaged when the inner member is in the second position.

According to the present invention, a fluid transfer device includes an inner member configured to be secured to a container and an outer member surrounding at least a portion of the inner member. The inner member having a first position where the inner member is configured to rotate freely relative to the outer member and a second position where rotation of the inner member relative to the outer member is restricted in a first rotational direction. The device further includes a seal arrangement positioned within the outer member. The seal arrangement is moveable within the outer member and configured to engage a mating connector. The seal arrangement having an actuated position adjacent to the inner member and an initial position spaced from the inner member, where the seal arrangement prevents the inner member from moving from the first position to the second position when the seal arrangement is in the actuated position.

The inner member and the outer member define a ratchet arrangement, where the ratchet arrangement is only engaged when the inner member is in the second position.

The device may further include a locking arrangement provided on at least a portion of the inner member and accessible through at least a portion of the outer member. The inner member may be moveable in an axial direction within the outer member between the first and second positions of the inner member. The locking arrangement may be a recess defined by the inner member, the recess having an engagement surface.

In another aspect of the present invention, a patient connector and packaging includes a patient connector having a body and at least one protrusion extending radially outward from the body, and packaging having an open first end, a second end, and a sidewall extending between the first and second ends. The packaging includes a plurality of guide protrusions positioned adjacent to the first end of the packaging. The at least one protrusion is configured to be received between the plurality of guide protrusions of the packaging.

### BRIEF DESCRIPTION OF THE DRAWINGS

**FIG.** 1 is a perspective view of a fluid transfer device and packaging.
**FIG. 2** is a perspective view of packaging for a fluid transfer device.
**FIG. 3** is a perspective view of a fluid transfer device.
**FIG. 4** is a front view of the packaging of **FIG. 2****.**
**FIG. 5** is a right side view of the packaging of **FIG. 2****.**
**FIG. 6** is a rear view of the packaging of **FIG. 2****.**
**FIG. 7** is a left side view of the packaging of **FIG. 2****.**
**FIG. 8** is a top view of the packaging of **FIG. 2****.**
**FIG. 9** is a bottom view of the packaging of **FIG. 2****.**
**FIG. 10** is a perspective cross-sectional view of the fluid transfer device of **FIG. 3****.**
**FIG. 11** is a cross-sectional view of the fluid transfer device of **FIG. 3****.**
**FIG. 12** is a cross-sectional view of the fluid transfer device and packaging of **FIG.** 1.
**FIG. 13** is a side view of a luer body of the fluid transfer device of **FIG. 3****.**
**FIG. 14** is a perspective view of the luer body of **FIG. 13****.**
**FIG. 15** **is** a perspective view of a body of the fluid transfer device of **FIG. 3****.**
**FIG. 16** is a perspective view of a cap of the fluid transfer device of **FIG. 3****.**
**FIG. 17** is a cross-sectional view of the cap of **FIG. 16****.**
**FIG. 18** is a perspective view of a patient connector and packaging.
**FIG. 19** is a perspective view of a patient connector.
**FIG. 20** is a perspective view of packaging for a patient connector.
**FIG. 21** is a cross-sectional view of the packaging of **FIG. 20****.**

### DETAILED DESCRIPTION

Further, for purposes of the description hereinafter, the terms "end", "upper", "lower", "right", "left", "vertical", "horizontal", "top", "bottom", "lateral", "longitudinal", and derivatives thereof shall relate to the disclosure as it is oriented in the drawing figures. The term "proximal" refers to the direction toward the center or central region of the device. The term "distal" refers to the outward direction extending away from the central region of the device. Specific dimensions and other physical characteristics related to the aspects disclosed herein are not to be considered as limiting. For the purpose of facilitating understanding of the disclosure, the accompanying drawings and description illustrate preferred aspects thereof, from which the disclosure, various aspects of its structures, construction and method of operation, and many advantages may be understood and appreciated.

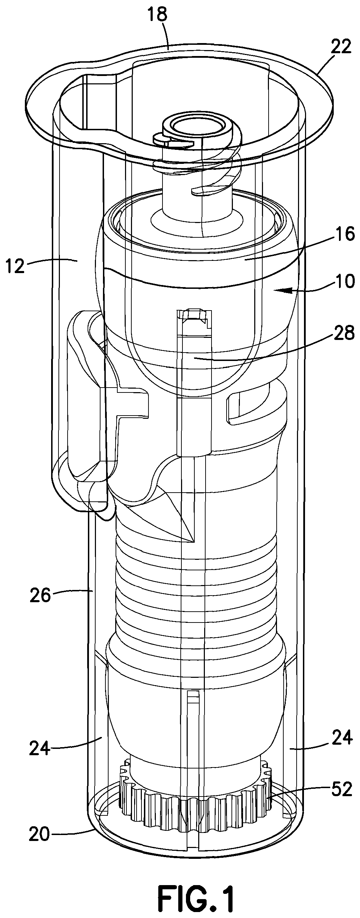

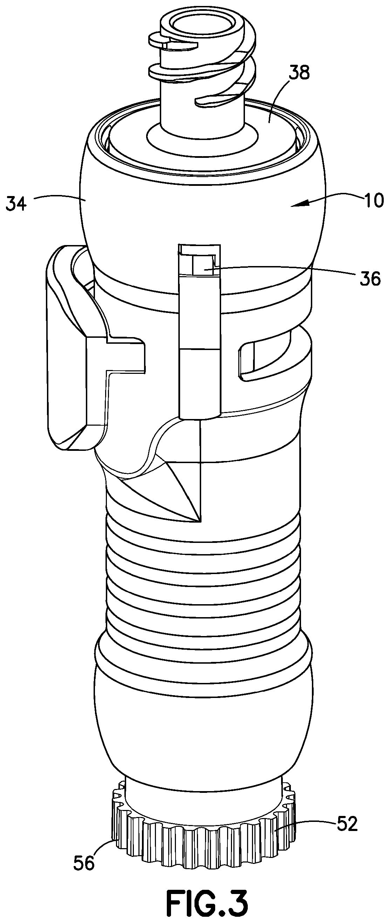

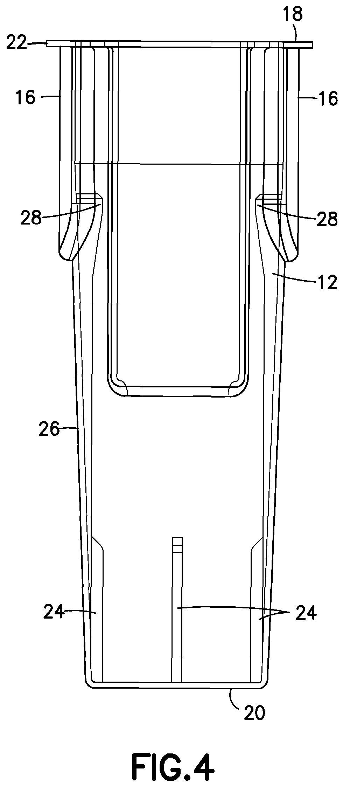

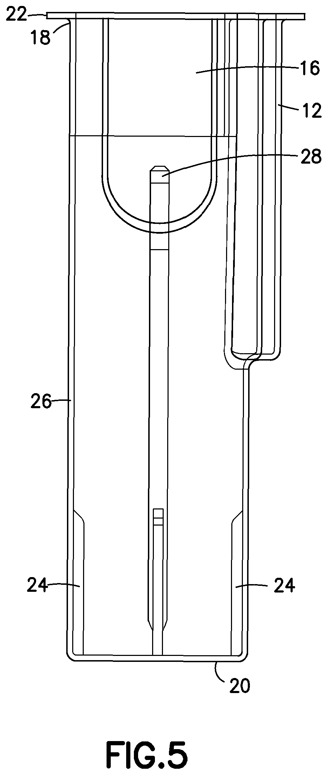

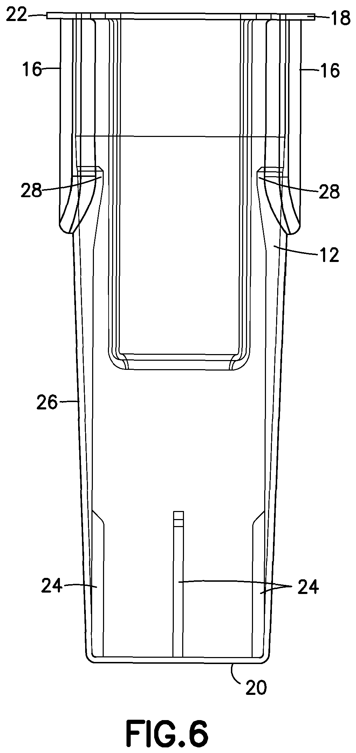

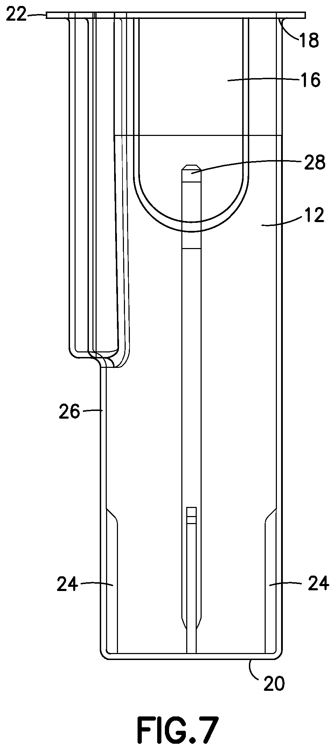

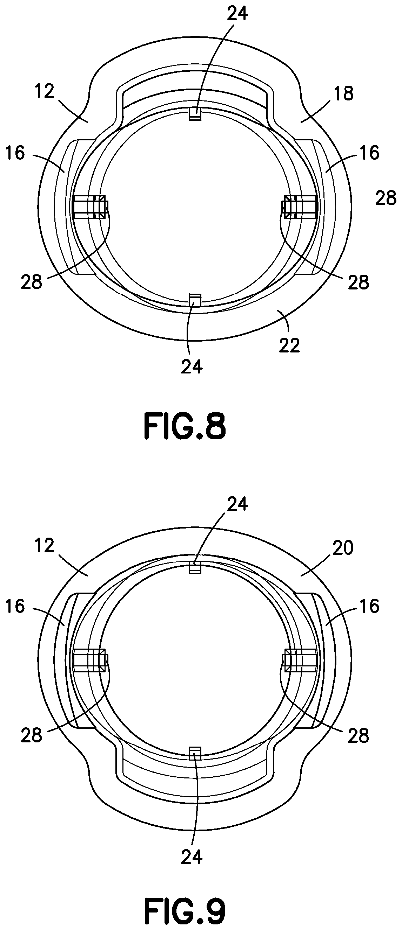

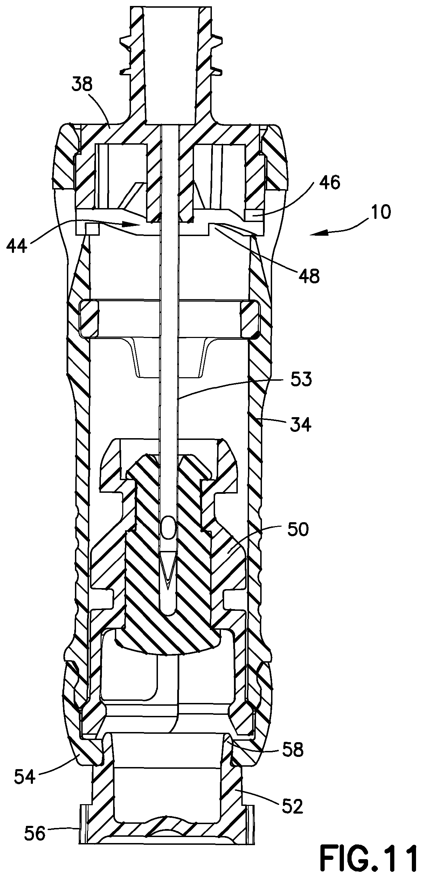

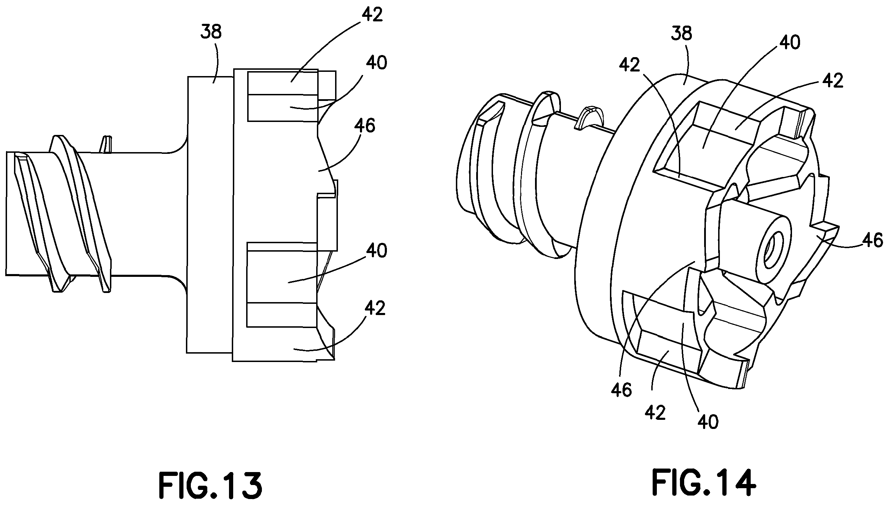

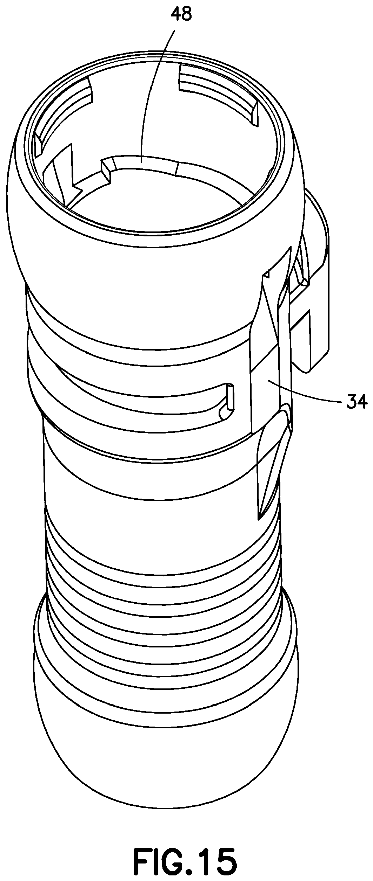

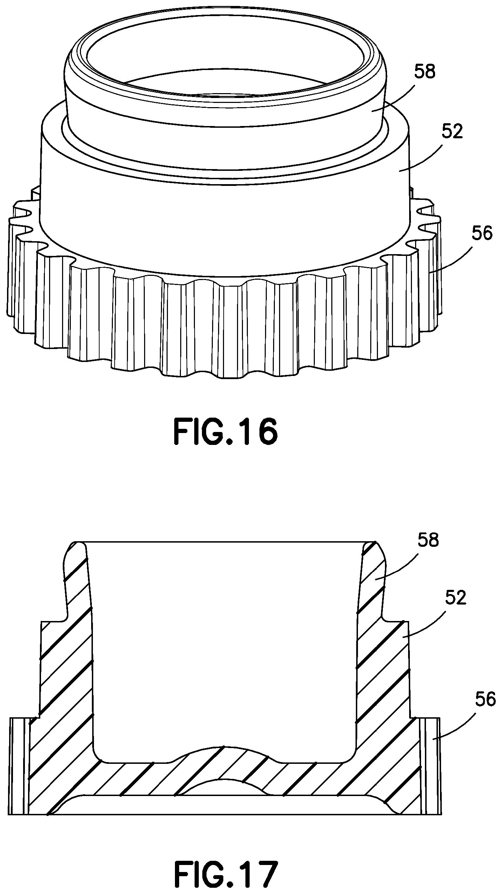

Referring to **FIGS. 1-17****,** a fluid transfer device **10** and packaging **12** for the fluid transfer device **10** is shown in accordance with one aspect of the invention. The fluid transfer device **10** facilitates the transfer of fluid between a first container (not shown), such as a syringe, and a second container, such as a vial, IV bag, IV line, etc. The fluid transfer device **10** facilitates such transfer while ensuring fluid does not leak from the fluid transfer device **10.** As discussed in more detail below, the packaging **12** holds the fluid transfer device **10** prior to use and functions to allow removal of a container, such as a syringe, from the fluid transfer device **10** after attachment thereto. In particular, as discussed below, the fluid transfer device **10** includes features that inhibit disconnection of a container from the fluid transfer device **10 to** prevent accidental leakage of fluid from the device **10** and the packaging **12** may be utilized to allow the intentional removal of a container from the fluid transfer device **10.**

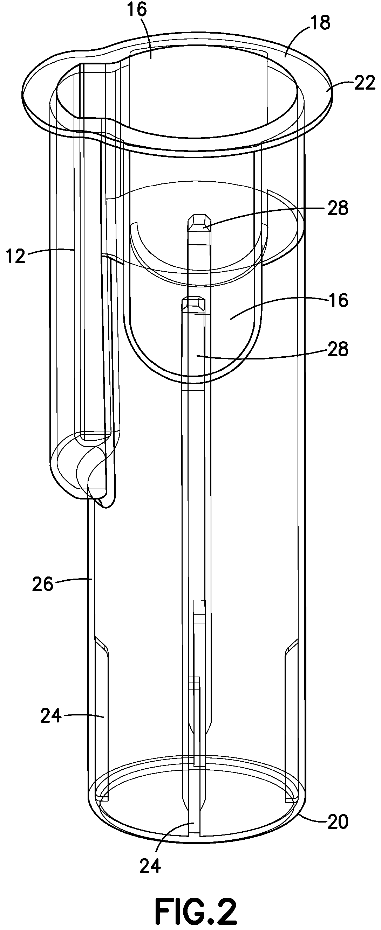

Referring to **FIGS. 1****,** **2****,** and **4****-9,** the packaging **12** is shown as transparent, although the packaging **12** may or may not be made from a transparent or semi-transparent material. The packaging **12** defines an interior cavity **14** and includes elongate buttons **16** that extend from a position intermediate first and second ends **18, 20** of the packaging **12** to a flange **22** at the first end **18** of the packaging **12.** The elongate buttons **16** provide a larger surface area for a user to engage during use the packaging **12** compared to circular buttons or tabs. As shown in **FIGS. 1** and **2****,** the packaging **12** also includes a plurality of ribs **24** protruding from a sidewall **26** of the packaging **12** radially inward. The plurality of ribs **24** extend longitudinally from the second end **20** of the packaging **12** towards the first end **18** of the packaging **12.** The plurality of ribs **24** are configured to engage a portion of the fluid transfer device **10** to prevent movement of the fluid transfer device **10** within the packaging **12** until a user purposefully removes the fluid transfer device **10** from the packaging **12.** In particular, the plurality of ribs **24** may prevent the fluid transfer device **10** from sliding axially within the packaging **12,** which can cause damage and wear to the closure (not shown) of the packaging **12.**

The packaging **12** also includes projections **28** extending radially inward from the sidewall **26** that are configured to move radially inward upon engagement of the elongate buttons **16.** The projections **28** extend in a direction substantially parallel to the longitudinal axis of the packaging **12.** In certain aspects, the projections **28** may be angled relative to the longitudinal axis. The projections **28** may have any desired shape, including, but not limited to, square, rectangular, rounded, etc. In one aspect, the projections **28** extend from a region of the inner sidewall **26** proximate to the closed bottom end **20** to an area of the inner sidewall **26** opposite the elongate buttons **16.** The projections **28** may be oriented 180 degrees apart around a circumference of the packaging **12** such that each projection **28** is aligned with the elongate button **16.** For example, the longitudinal midpoint of each projection **28** may be aligned with an axis extending through the center of each elongate button **16.** The projections **28** may define an alignment feature for aligning the fluid transfer device **10** within the interior cavity **14** of the packaging **12.** The projections **28** are configured to deflect radially inward in response to a radially-directed force imparted on the elongate buttons **16.** Although the projections **28** are separated equally about the circumference of the packaging **12,** it is to be appreciated that more than two projections **28** may be provided with equal or unequal separation about the circumference of the container **10.**

The packaging **14** may be constructed from any known material, such as a molded, injected, or thermo-formed plastic material. Desirably, the packaging **14** is constructed from a material that provides flexibility of the sidewall **26** in at least the radial direction with respect to the longitudinal axis. In particular, the packaging **14** is desirably constructed from a material that allows the cross-sectional shape of the packaging **14** to change with an application of a radially-directed force, as will be described in greater detail below.

Referring to **FIGS. 1****,** **2****,** and **4****-9,** the packaging **12** further includes a recess **32** that is configured for receiving an activation tab of the fluid transfer device **10,** as will be described hereinafter. The recess **32** extends radially outward relative to the longitudinal axis of the packaging **12.** The recess **32** also extends along at least a portion of the longitudinal length of the packaging **12.** The recess **32** is shaped such that the sidewall **26** bulges radially outward in the area of the recess **32.** In addition to accommodating a portion of the fluid transfer device **10,** the recess **32** also orients the fluid transfer device **10** such that it can be received in the interior cavity **14** in one direction only. Other features of the fluid transfer device **10** or the packaging **12** may be used to align the fluid transfer device **10** within the interior cavity **14** of the container **10.**

Referring to **FIGS. 1****,** **3****,** and **10****-17,** the fluid transfer device **10** includes an outer member **34** defining openings **36** that are configured to receive at least a portion of the projections **28** of the packaging **12.** Further, the fluid transfer device **10** includes an inner member **38** having recesses **40** and engagement surfaces **42** that are configured to cooperate with the projections **28** of the packaging **12** to restrict relative movement of the inner member **38** relative to the outer member **34** as discussed in more detail below. The inner member **38** is embodied as a luer body, although any other suitable connection arrangements may be utilized. The fluid transfer device **10** also includes a ratchet arrangement **44** formed by portions of the inner member **38** and the outer member **34.** The inner member **38** is moveable in an axial direction relative to the outer member **34** and includes ratchet teeth **46** configured to cooperate with corresponding shaped projections **48** on the outer member **34.** The inner member **38** has a first axial position where the inner member **38** is free to rotate relative to the outer member **34** and a second axial position where the ratchet teeth **46** are engaged with the projections **48** of the outer member **34** to restrict relative movement between the inner member **38** and the outer member **34** in a single rotational direction. Thus, when the inner member **38** is in the second axial position, the inner member **38** will be able to rotate relative to the outer member **34** is one rotational direction, but restricted from moving relative to the outer member **34** is an opposite rotational direction.

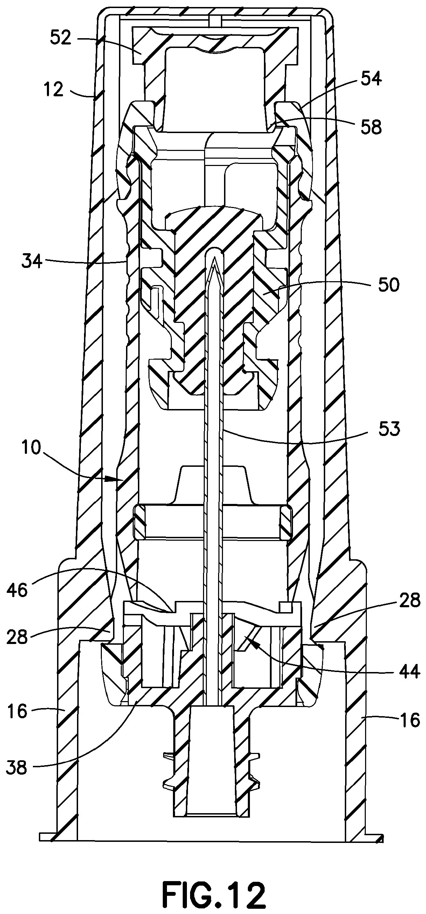

Referring to **FIGS. 1** and **12****,** in order to attach a container, such as a syringe barrel, to the inner member **38,** an axial force must be applied to the inner member **38** to move the inner member **38** from the first position to the second position, which will engage the ratchet arrangement **44** to prevent relative rotation between the inner and outer members **38, 34** to allow the container to be secured to the inner member **38.** During this attachment, a portion of the projections **28** of the packaging **12** will contact a portion of the outer member **34** to provide resistance to torque created by the attachment to prevent rotation of the fluid transfer device **10** relative to the packaging **12.** Any portion of the fluid transfer device **10** may interface with a corresponding portion of the packing **12** to prevent rotation of the fluid transfer device **10** relative to the packaging **12** to readily allow attachment to the inner member **38.**

The axial force may be a slight nudge of the inner member **38,** although any other suitable axial force may be utilized. Rotation in an opposite direction to remove the container from the fluid transfer device **10,** however, will not engage the ratchet arrangement **44** and the inner member **38** will merely rotate within the outer member **34** due to the sloped surface of the ratchet teeth **46.** Thus, a container cannot be removed from the inner member **38** of the fluid transfer device **10** when the inner member **38** is in the first or second position without the use of the packaging **12.** The container can be removed from the fluid transfer device **10** by inserting the fluid transfer device **10** into the packaging **12** and engaging the elongate buttons **16** of the packaging **12** such that the projections **28** of the packaging **12** are received by the engagement surfaces **42** to lock the inner member **38** relative to the outer member **34.** With the inner member **38** locked or rotationally fixed relative to the outer member **34,** the container can be removed from the inner member **38** of the fluid transfer device **10.**

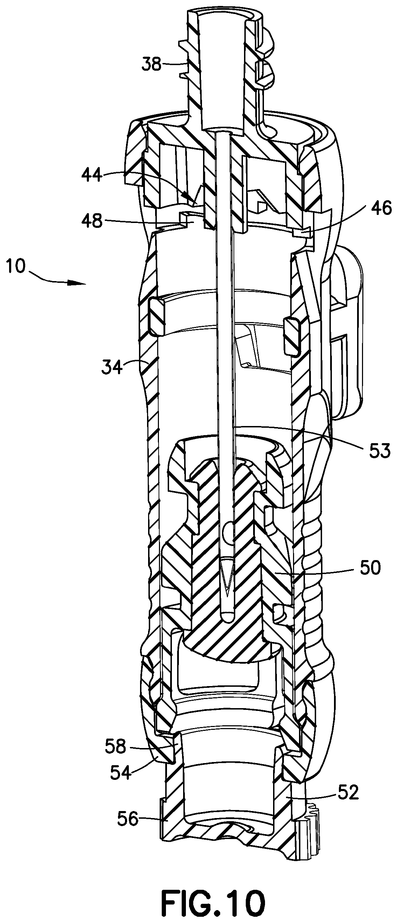

Referring to **FIGS. 10-12****,** the fluid transfer device **10** includes a seal arrangement **50,** such as a collet and membrane assembly shown in **FIGS. 10-12****,** that cooperates with a mating connector and moves within the outer member **34** to provide for the sealed transfer of fluids using the fluid transfer device **10** as discussed above. The fluid transfer device **10** also include a cannula **53** secured to the inner member **38** that is configured to pierce the membrane of the seal arrangement **50** and the membrane of the mating connector during the sealed transfer of fluids using the device **10.** The seal arrangement **50** also secures the fluid transfer device **10** to the mating connector or component during the transfer of fluids. The fluid transfer device **10** may further include a secondary arrangement for ensuring the device **10** is secured to the mating connector or component during fluid transfer. In particular, the fluid transfer device may include an activation tab **51** provided in an opening of the outer member **34** and extending perpendicularly through the outer member **34.** The activation tab **51** cooperates with the seal arrangement **50** and is secured to the seal arrangement **50** when the seal arrangement **50** is in an actuation position adjacent to the inner member **38** to maintain the seal arrangement **50** in that position. In such a position, the mating connector or component that is mated with the seal arrangement **50** cannot be disconnected from the fluid transfer device **10.** The mating connector or component can be disengaged from the fluid transfer device **10** by pushing or engaging the activation tab **51** radially inward, which allows the mating connector to move the seal arrangement **50** thereby allowing the mating connector to be withdrawn from the outer member **34** of the fluid transfer device **10.** Further, the activation tab **51** may interface with the recess **32** of the packaging **12** to prevent rotation of the fluid transfer device **10** relative to the packaging **12** while attachment of the container to the inner member **38** is made.

Referring again to **FIGS. 10-12****,** in one aspect of the present invention, the fluid transfer device **10** also includes a lift-off feature that restricts the movement of the inner member **38** from the first position to the second position. In one position, such as when the fluid transfer device **10** is connected to a mating connector or component, the seal arrangement **50** is in an actuation position adjacent to the inner member **38.** In this position, the seal arrangement **50** prevents the inner member **38** from moving from the first position to the second position described above. Accordingly, the seal arrangement **50** prevents the ratchet arrangement **44** from being engaged. In other words, the seal arrangement **50** abuts the inner member **38** to prevent the inner member **38** from moving to the second position where the ratchet teeth **46** can engage the projections **48.** Although the ratchet arrangement **44** only allows for tightening of a container, such as a syringe, onto the inner member **38,** an alternative arrangement may be provided that allows disconnection and/or connection of a syringe to the inner member **38** when the inner member **38** is in the second position, which can be deactivated via the lift-off feature described above.

Referring to **FIGS. 1****,** **10-13****,** **17****,** and **18****,** the fluid transfer device **10** also includes a cap 52 configured to close an end **54** of the fluid transfer device **10.** The cap **52** includes a gripping surface **56** and a tapered insertion end **58** that is received by the fluid transfer device **10.** The tapered insertion end **58** may deflect radially inward upon insertion into the fluid transfer device **10** to secure the cap **52** to the fluid transfer device **10,** although other suitable arrangements for securing the cap **52** to the fluid transfer device **10** may be utilized. The gripping surface **56** provides a surface for a user to engage to remove the cap **52** from the fluid transfer device **10** prior to use.

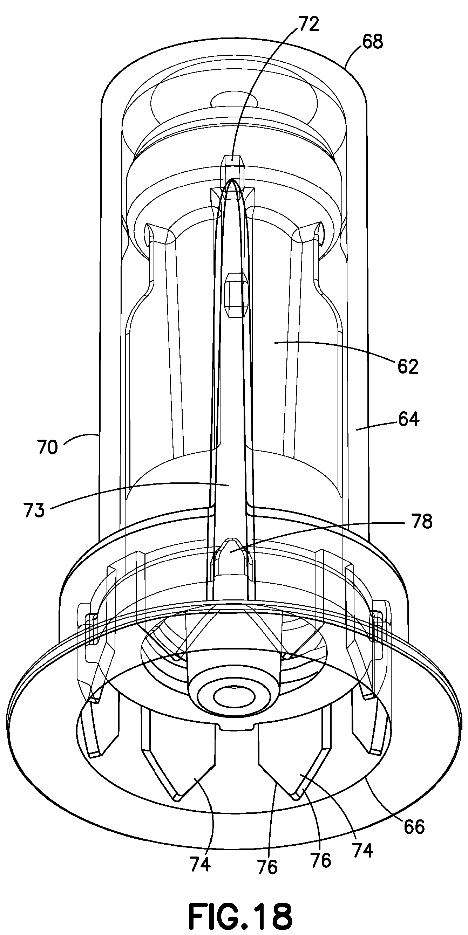

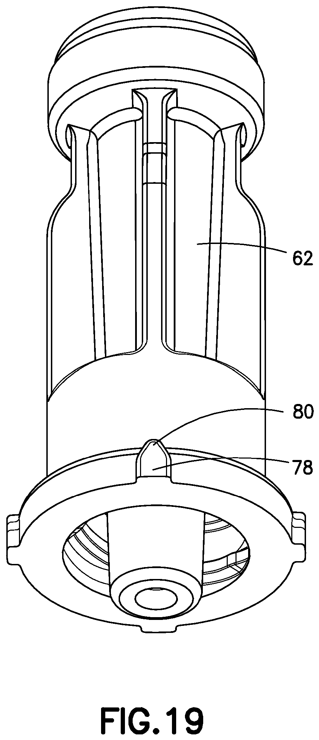

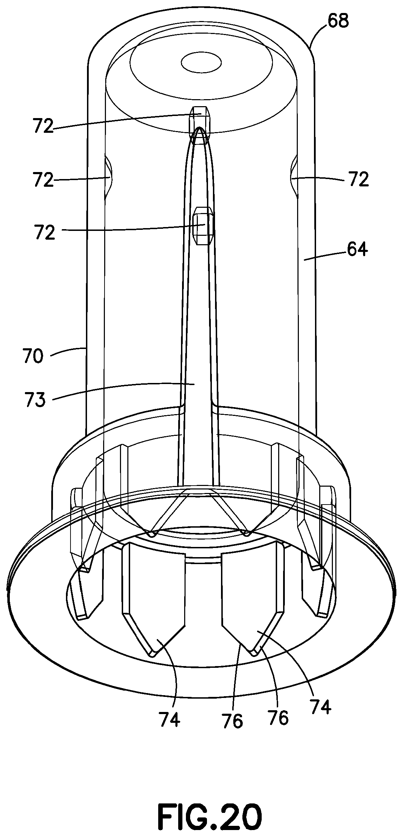

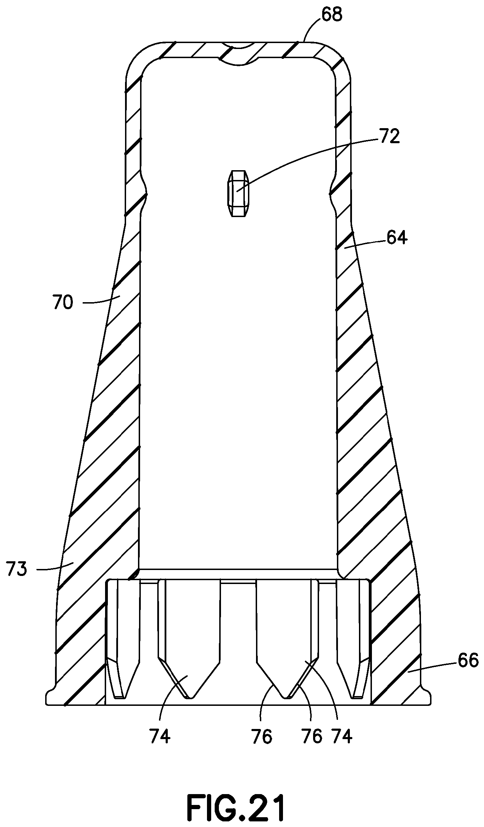

Referring to **FIGS. 18-21****,** a patient connector **62** and packaging **64** for the patient connector **62** is shown in accordance with one aspect of the present invention. The packaging **64** is shown as transparent in **FIGS. 18-21****,** although the packaging **64** may or may not be made from a transparent or semi-transparent material. The packaging **64** has an open first end **66** and a second end **68** with a sidewall **70** extending between the first and second ends **66, 68.** The open first end **66** of the packaging **64** is closed by a removable closure (not shown). The packaging **64** includes a plurality of ribs **72** extending radially inward adjacent to the second end **68** of the packaging **64** that are configured to engage the patient connector **62** and restrict movement of the patient connector **62** within the packaging **64.** Further, the packaging **64** includes external ribs **73** that extend radially outward from the sidewall **70** of the packaging **64,** which may provide additional structural support and/or an additional gripping surface for the packaging **64.** The packaging **64** also includes a plurality of guide protrusions **74** positioned adjacent the first end **66** of the packaging **64.** The guide protrusions **74** include tapered surfaces **76** that are configured to guide a portion of the patient connector **62** into the packaging **64.** In particular, the patient connector **62** includes a plurality of protrusions **78** extending radially outward from the patient connector **62** that are configured to be received between the plurality of guide protrusions **74** of the packaging **64** with the tapered surfaces **76** of the packaging **64** cooperating with tapered surfaces **80** of the plurality of protrusions **78** of the patient connector **62** to guide insertion of the patient connector **62** into the packaging **64.** The positioning of the plurality of protrusions **78** between the guide protrusions **74** of the packaging **64** restricts rotational movement of the patient connector **62** relative to the packaging **64** to prevent wear and damage to the packaging **64** and patient connector **62** and also to facilitate connection of the patient connector **62** to a patient line (not shown). In other words, a patient line may be secured to the patient connector **62** while the patent connector **62** is positioned within the packaging **64,** which is easier with the patient connector **62** rotationally fixed relative to the packaging **64.**

## Claims

1. A fluid transfer device (10) comprising:
an inner member (38) configured to be secured to a container; and
an outer member (34) surrounding at least a portion of the inner member (38), wherein the inner member (38) is movable in an axial direction within the outer member (34) between a first axial position and a second axial position,
wherein:
the fluid transfer device (10) comprises a ratchet arrangement (44) in which the inner member (38) comprises ratchet teeth (46) configured to cooperate with corresponding shaped projections (48) on the outer member (34), such that in the first axial position the inner member (38) is free to rotate relative to the outer member (34), and in the second axial position, the ratchet teeth (46) of the inner member (38) are engaged with the projections (48) of the outer member (34) to restrict relative movement between the inner member (38) and the outer member (34) in a single rotational direction, and
the fluid transfer device (10) further comprises a seal arrangement (50) positioned within the outer member (34), the seal arrangement (50) is moveable within the outer member (34) and configured to engage a mating connector, the seal arrangement (50) having an actuated position adjacent to the inner member (38) and an initial position spaced from the inner member (38), wherein the seal arrangement (50) prevents the inner member (38) from moving from the first axial position to the second axial position when the seal arrangement (50) is in the actuated position.

2. The fluid transfer device (10) of claim 1, wherein the ratchet arrangement (44) is only engaged when the inner member (38) is in the second axial position.

3. The fluid transfer device (10) of claim 1, further comprising a locking arrangement provided on at least a portion of the inner member (38) and accessible through at least a portion of the outer member (34).

4. The fluid transfer device (10) of claim 3, wherein the locking arrangement comprises a recess defined by the inner member (38), the recess (40) having an engagement (42) surface.

## Patentansprüche

1. Fluidtransfervorrichtung (10), die aufweist:
ein Innenelement (38), das dazu ausgebildet ist, an einem Behälter befestigt zu werden; und
ein Außenelement (34), das zumindest einen Teil des Innenelements (38) umgibt, wobei das Innenelement (38) innerhalb des Außenelements (34) in axialer Richtung zwischen einer ersten axialen Position und einer zweiten axialen Position bewegbar ist,
wobei:
die Fluidtransfervorrichtung (10) eine Sperrklinkenbaugruppe (44) aufweist, bei der das Innenelement (38) Sperrklinkenzähne (46) aufweist, die so ausgebildet sind, dass sie mit entsprechend geformten Vorsprüngen (48) am Außenelement (34) zusammenwirken, sodass sich das Innenelement (38) in der ersten axialen Position frei relativ zum Außenelement (34) drehen kann, und in der zweiten axialen Position die Sperrklinkenzähne (46) des Innenelements (38) mit den Vorsprüngen (48) des Außenelements (34) in Eingriff stehen, um die relative Bewegung zwischen dem Innenelement (38) und dem Außenelement (34) in einer einzelnen Drehrichtung zu begrenzen, und
die Fluidtransfervorrichtung (10) ferner eine Dichtungsbaugruppe (50) aufweist, die innerhalb des Außenelements (34) angeordnet ist, wobei die Dichtungsbaugruppe (50) innerhalb des Außenelements (34) bewegbar ist und so ausgebildet ist, dass sie mit einem passenden Verbindungsstück in Eingriff kommt, wobei die Dichtungsbaugruppe (50) eine betätigte Position neben dem Innenelement (38) und eine vom Innenelement (38) beabstandete Ausgangsposition aufweist, wobei die Dichtungsbaugruppe (50) verhindert, dass sich das Innenelement (38) von der ersten axialen Position in die zweite axiale Position bewegt, wenn sich die Dichtungsbaugruppe (50) in der betätigten Position befindet.

2. Fluidtransfervorrichtung (10) nach Anspruch 1, wobei die Sperrklinkenbaugruppe (44) nur dann in Eingriff steht, wenn sich das Innenelement (38) in der zweiten axialen Position befindet.

3. Fluidtransfervorrichtung (10) nach Anspruch 1, die ferner eine Verriegelungsbaugruppe aufweist, die an zumindest einem Teil des Innenelements (38) vorgesehen und durch mindestens einen Teil des Außenelements (34) zugänglich ist.

4. Fluidtransfervorrichtung (10) nach Anspruch 3, wobei die Verriegelungsbaugruppe eine durch das Innenelement (38) begrenzte Aussparung aufweist, wobei die Aussparung (40) eine Eingriffsfläche (42) aufweist.

## Revendications

1. Dispositif de transfert de fluide (10) comprenant :
un élément interne (38) configuré pour être fixé à un récipient ; et
un élément externe (34) entourant au moins une partie de l'élément interne (38), dans lequel l'élément interne (38) est mobile dans une direction axiale à l'intérieur de l'élément externe (34) entre une première position axiale et une seconde position axiale,
dans lequel :
le dispositif de transfert de fluide (10) comprend un agencement à cliquet (44) dans lequel l'élément interne (38) comprend des dents à cliquet (46) configurées pour coopérer avec des saillies (48) de forme correspondante sur l'élément externe (34), de sorte que dans la première position axiale l'élément interne (38) soit libre de tourner par rapport à l'élément externe (34), et dans la seconde position axiale, les dents à cliquet (46) de l'élément interne (38) soient en prise avec les saillies (48) de l'élément externe (34) pour restreindre le mouvement relatif entre l'élément interne (38) et l'élément externe (34) dans une seule direction de rotation, et
le dispositif de transfert de fluide (10) comprend en outre un agencement d'étanchéité (50) positionné à l'intérieur de l'élément externe (34), l'agencement d'étanchéité (50) est mobile à l'intérieur de l'élément externe (34) et configuré pour être en prise avec un connecteur d'accouplement, l'agencement d'étanchéité (50) présentant une position actionnée adjacente à l'élément interne (38) et une position initiale espacée de l'élément interne (38), dans lequel l'agencement d'étanchéité (50) empêche l'élément interne (38) de se déplacer de la première position axiale à la seconde position axiale lorsque l'agencement d'étanchéité (50) est dans la position actionnée.

2. Dispositif de transfert de fluide (10) selon la revendication 1, dans lequel l'agencement à cliquet (44) n'est en prise que lorsque l'élément interne (38) est dans la seconde position axiale.

3. Dispositif de transfert de fluide (10) selon la revendication 1, comprenant en outre un agencement de verrouillage prévu sur au moins une partie de l'élément interne (38) et accessible à travers au moins une partie de l'élément externe (34).

4. Dispositif de transfert de fluide (10) selon la revendication 3, dans lequel l'agencement de verrouillage comprend un évidement défini par l'élément interne (38), l'évidement (40) présentant une surface de mise en prise (42).
